# EUROPEAN PATENT SPECIFICATION

(11) **EP 3 673 946 B1**
(45) Date of publication and mention of the grant of the patent: **19.10.2022**
(21) Application number: 18885883.1
(22) Date of filing: 03.01.2018
(51) Int. Cl.: A61M 25/09, A61B 5/03, A61B 5/00, A61B 5/0215

(54) **PRESSURE GUIDE WIRE**
DRUCKFÜHRUNGSDRAHT
FIL-GUIDE DE PRESSION

(30) Priority: 07.12.2017 CN 201711284145
(43) Date of publication of application: 01.07.2020
(73) Proprietor: Innovex Medical Co., Ltd., Shanghai 201210 (CN)
(72) Inventor: ZHENG, Zhongwei, Shanghai 201210 (CN); YAN, Hang, Shanghai 201210 (CN); WANG, Pan, Shanghai 201210 (CN); YUAN, Qing, Shanghai 201210 (CN); CHEN, Qi, Shanghai 201210 (CN)
(74) Representative: Zaboliene, Reda
(86) International application number: PCT/CN2018/070098
(87) International publication number: WO 2019/109442

(56) References cited:
- WO-A1-02/085442
- WO-A1-2014/140883
- WO-A2-00/38567
- CN-A- 103 328 033
- CN-A- 106 214 140
- CN-U- 204 445 869
- CN-U- 206 414 602
- CN-U- 206 414 602
- US-A1- 2003 028 128
- US-A1- 2005 268 725
- US-A1- 2016 121 085
- US-A1- 2016 303 354
- US-A1- 2016 303 354
- US-B2- 7 724 148

## Description

### Field of the invention

The present invention relates to the field of medical device technology, and more particularly to a pressure guide wire.

### Background of the Invention

Since non-vascular systems such as urinary, digestive, respiratory, and reproductive systems are connected to the outside of the human body, the minimally invasive endoscopic surgery today is often the choice of treatment of non-vascular diseases. For example, cystoscopy, ureteroscopy, nephroscope and other endoscopes are used in the urinary system for surgical treatment. During the treatment process, it is necessary to connect the medical perfusion fluid through the water injection channel on the endoscope to maintain the clarity of the surgical field of vision and remove the extras generated by the surgery. However, the current pressure of the perfusion fluid is mainly controlled by the experience of the operator, it is easy to cause a large amount of perfusion fluid into the blood if the pressure in the human urinary system is too high. The perfusion fluid contains a large amount of bacterial endotoxins, which will affect the human circulatory system after entering the blood, causing hypothermia, electrolyte disturbances, blood biochemistry, and electrodynamic changes in the body, further causing bacteremia, endotoxemia, and septic shock occurred, and threatening the lives of patients seriously. Therefore, real-time monitoring and precise control of the actual pressure changes in the human urinary system are particularly important.

A variety of non-invasive pressure measurement techniques used in non-vascular systems have been disclosed in the prior art. For example, references CN205107661U and CN204428509U has both disclosed a pressure device for the urinary system, but both of them place the hose into the human body , then measure by connecting the pressure sensor to the other end of the hose at the outside of the body based on the principle of the connector. The in vitro measurement method has the disadvantages of poor sensitivity, low precision, many interference factors, and unstable measurement results. Distortion or even measurement failure of measured values may be caused by micro-bubbles in the pipeline, jitter during surgery, debris clogging after stone crushing, etc. can cause.

The importance of providing an in intracorporeal pressure measurement device has become increasingly prominent due to the various problems described above in in vitro pressure measurement. Reference CN104146699A discloses an intrarenal pressure monitoring and control system, comprising a fiber optic pressure sensor, a signal tuner, a signal splitter, a display, a feedback controller and a medical perfusion pump. The fiber optic pressure sensor is inserted into the kidney through the operating hole of the surgical endoscope, and is connected to the signal tuner through the optical fiber, which is used for real-time pressure monitoring of the intraoperative operation area of the kidney cavity. The signal tuner is connected to the signal input of the signal splitter, the signal output of the signal splitter is respectively connected to the display and feedback controller, and the feedback controller is connected to the medical perfusion pump. The medical perfusion fluid is injected into the medical perfusion pump through the catheter and is injected into the kidney cavity through the catheter under the control of the medical perfusion pump. The technical solution can accurately measure the real-time pressure in the kidney and feed it back to the medical perfusion pump, which ensures the safety of various intrarenal operations. However, the technical solution only provides a concept of intrarenal pressure monitoring, which does not disclose an intrarenal pressure measurement device with a specific structure.

For a specific intracorporeal pressure measurement device, various pressure measuring guide wires used in blood vessels have been disclosed in the prior arts. The blood pressure of the blood vessels in the living body is obtained based on the pressure signal of the pressure sensor by installing a pressure sensor at the distal end of the guide wire, then the FFR value is calculated to evaluate the blood flow level of the blood vessel, and finally the degree of vascular stenosis is judged functionally. However, since the guide wire is applied to the blood vessels, in general, the blood vessels are relatively narrow, and the path is relatively curved and complex, the pressure measuring guide wire used in blood vessels generally hollow out the mandrel, the pressure sensor is arranged inside the mandrel, and a large opening is provided on the mandrel of the corresponding area of the sensor, so that the cavity in which the pressure sensor is located is connected to the outside, and the intravascular pressure is measured on the basis of avoiding direct contact between the pressure sensor and the vessel wall. But the measurement accuracy of such a pressure sensor is greatly affected by the position and the size of the opening , which is not easy to process, while there is also a high degree of difficulty in product design and assembly, and the operation convenience in the manufacturing process is extremely poor.

In addition, the reference CN103720463A disclosed a pressure guide wire used in cardiovascular intervention therapy. The main body of the pressure guide wire is a solid structure, and multi-point distributed pressure sensors are provided at the end of the pressure guide wire. The pressure sensors are connected to the internal lead wire and go straight to the head along the guide wire, and the head is equipped with sensor lead electrode points, which are connected to the control handle to collect the blood pressure signal measured by the pressure sensors in real time. The pressure sensors have the characteristics of full flexibility and are wrapped around the surface of the guide wire in a spiral manner. The guide head is designed to be flat, which is convenient to connect with an external torque sensor on the control handle to measure the feedback torque when the guide wire rotates in real time. The pressure sensor and the sensing lead wire of the technical solution are all fixed in a fully flexible film to obtain a flexible sensor layer, the flexible sensor layer is uniformly attached to the set position of the core by means of spiral winding after applying the adhesive on the core of the guide wire. However, the flexibility of the guide wire is greatly affected due to the high hardness after the adhesive is dried, and the possibility of cracking and separation of the flexible film and the mandrel is easy to occur during the bending process of the guide wire. In addition, a gap is easily formed between each spirally wound flexible film, which causes the surface of the guide wire to be uneven and damage to the inner wall of the inserted cavity, thereby endangering human health. Patent document WO 2014/140883 A1 discloses a pressure guide wire for use in a vascular system.

Therefore, the urgent problem to be solved in the medical field is to provide a pressure guide wire which can be used in a non-vascular system with high measurement accuracy and simple processing on the basis of the above-mentioned prior art.

### SUMMARY OF THE INVENTION

The technical problem to be solved by the present invention is to provide a pressure guide wire which can be used in a non-vascular system with high measurement accuracy and simple processing.

In order to solve the above-mentioned technical problems, the present invention provides a pressure guide wire according to claim 1.

Further according to the invention, the operation portion transmits the signal of the pressure sensor to the external display device by wire or wireless means.

Further, according to an embodiment, the portions of the plurality of sensing wires located between the proximal-end protective sleeve and the mandrel do not cross each other.

Further, according to an embodiment, the portions of the plurality of sensing wires located between the proximal-end protective sleeve and the mandrel is uniformly distributed along the circumferential direction on the surface of the mandrel.

Further, according to an embodiment, the portions of the plurality of sensing wires located between the proximal-end protective sleeve and the mandrel is spirally wound on the surface of the mandrel.

Further, according to an embodiment, the pressure sensor is adhered by adhesive, or fixed by a physical structure in the groove of the intermediate ring.

Further, according to an embodiment, the proximal-end protective sleeve is a heat shrink tube, and the heat shrink tube is made of a material selected from fluoropolymer, polyurethane, and nylon elastomer.

Further, according to an embodiment, the heat shrink tube is made of a fluorinated ethylene propylene copolymer. Further, the outer diameter of the distal end of the intermediate ring and the proximal end of the distal-end protective sleeve are substantially the same; the outer diameter of the proximal end of the intermediate ring and the distal end of the proximal-end protective sleeve are substantially the same.

Further, according to an embodiment, the intermediate ring is made of a metal material.

Further, according to an embodiment, the distal end of the proximal-end protective sleeve and/or the distal-end protective sleeve is further wrapped with a functional coating layer, the functional coating layer is a hydrophilic coating, a lubricating coating or a bio-coating.

In summary, by fixing the pressure sensor in the groove of the intermediate ring, that is in the groove of the intermediate sleeve of claim 1, the present invention can make the pressure sensor directly contact the liquid in the human body's lumen, so that the pressure change in the human body's lumen can be monitored accurately and in real time; the sensing wires is wrapped by the proximal-end protective sleeve ,and there is no adhesive between the proximal-end protective sleeve and the mandrel, so the flexibility of the guide wire is good, the guide wire will not crack when bent, and the service life is long, the durability is good. In addition, the entire proximal-end protective sleeve is used to wrap the sensing wires, so the processing is simple, the outer wall of the guide wire is smooth, which avoids scratching human organs and has good safety.

### BRIEF DESCRIPTION OF THE DRAWINGS

The present invention will be further described in detail below with reference to the accompanying drawings and specific embodiments:
Fig.1 is the schematic structural view of the guide wire portion of the pressure guide wire according to the present invention,
Fig.2 is the cross-sectional view taken along line A-A of Fig. 1,
Fig.3 is the schematic structural view of the sensing wire spirally wound on the mandrel according to the present invention;
Fig.4 is the schematic structural view of the intermediate ring according to the present invention;
Fig.5 is the schematic structural view showing the connection mode of the pressure guide wire and the display device according to the present invention.
Fig.6 is the schematic structural view showing another connection mode of the pressure guide wire and the display device according to the present invention.

The component numbers are as follows:
1- distal-end protective sleeve
2- intermediate ring
21- groove
3- proximal-end protective sleeve
4- mandrel
5- pressure sensor
51- sensing wire
6- operation portion
7- display device

### DETAILED DESCRIPTION OF THE INVENTION

Preferred embodiments of the present invention will now be described in detail in conjunction with the drawings. Although the description of the present invention will be described in conjunction with the various embodiments, it is not intended that the features of the invention are limited to the embodiments. Rather, the invention, which is defined by the appended claims, is described in connection with the embodiments so as to cover other alternatives or modifications that are possible in the embodiments of the invention. In order to provide a thorough understanding of the present invention, many specific details are included in the following description. The invention may also be practiced without these details. In addition, some specific details are omitted from the description in order to avoid confusion or blur the focus of the present invention.

In addition, "upper", "lower", "left", "right", "top", and "bottom" used in the following description are set to better describe the preferred embodiment of the present invention. It should not be construed as limiting the invention.

As used herein, "proximal end" or "tail end" is the end closest to the physician in use. The "distal end" or "front end" is the end of the body cavity that is the farthest from the entrance, that is, the end farthest from the doctor or the insertion end.

In the following drawings, the full length of the guide wire is not shown. The length of the guide wire can be changed according to the requirements, but typically the length of the guide wire is in the range of 30 to 800 centimeters (cm).

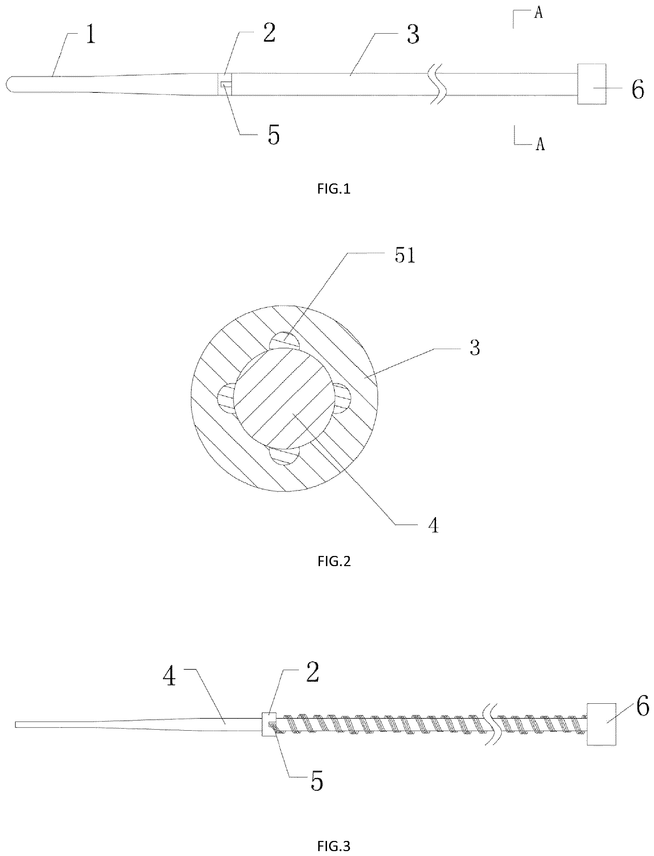

The technical solution of the present invention is shown in FIGS. 1 to 6, the guide wire comprises an insertion portion and an operation portion 6. The insertion portion comprises a mandrel 4, a distal-end protective sleeve 1 wrapping the distal end portion of the mandrel 4, a proximal-end protective sleeve 3 wrapping the proximal end portion of the mandrel 4, an intermediate ring 2 disposed between the distal-end protective sleeve 1 and the proximal-end protective sleeve 3, which is fixedly sleeved on the mandrel 4 and the outer surface of which is provided with a groove 21, and, a pressure sensor 5 disposed in the groove 21 of the intermediate ring 2, which connects with a plurality of sensing wires 51 extending between the proximal-end protective sleeve 3 and the mandrel 4, wherein the other end of the sensing wires 51 is connected to the operation portion 6. The operation portion 6 is connected to the proximal end of the insertion portion, which is used to transmit signals of the sensor to an external display device 7.

The present invention can make the pressure sensor 5 directly contacts the liquid in the human body's lumen by fixing the pressure sensor 5 in the groove 21 of the intermediate ring 2, so that the pressure change in the lumen can be monitored accurately and in real time. The sensing wires 51 is wrapped by the proximal-end protective sleeve 3 without adhesive between the proximal-end protective sleeve 3 and the mandrel 4, thus the flexibility of the guide wire is good, the guide wire will not crack when bent, and the durability is good. In addition, the proximal-end protective sleeve 3 is used to wrap the sensing wires 51 entirely, so the processing is simple and the outer wall of the guide wire is smooth, which can avoid scratching human organs and has good safety.

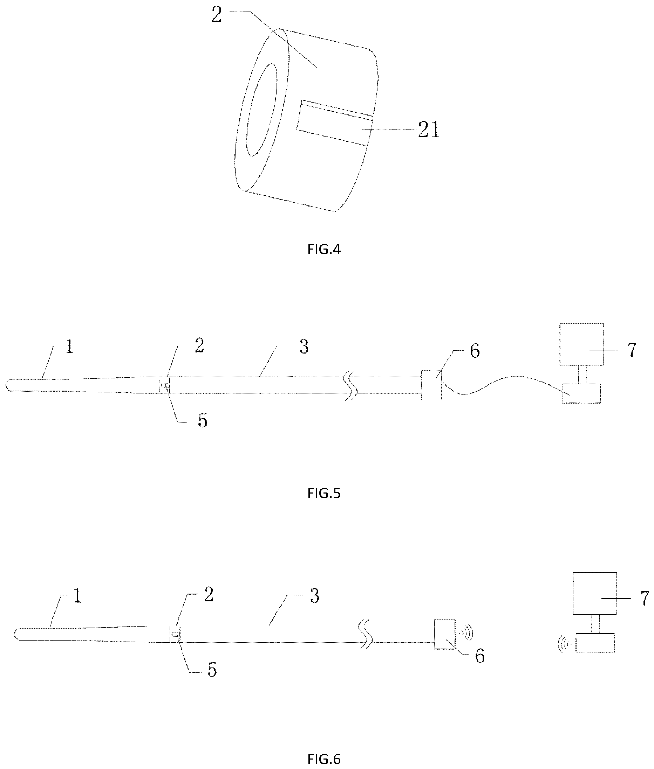

Further, the operation portion 6 transmits the signal of the pressure sensor 5 to the external display device 7 by wire or wireless means. When the wired connection method shown in FIG. 5 is adopted, the operation portion 6 is provided with a connection port, such as a common USB interface or a PS/2 interface. and the external display device 7 may be a mobile phone, a tablet computer or a computer, etc., by which an APP or a software program as a display program is loaded, and the external display device 7 and the operation portion 6 can be connected by a data cable.

Further, the external display device 7 and the operation portion 6 can also be connected by a wireless connection as shown in FIG. 6. At this time, a wireless communication module needs to be provided in the operation portion 6 for transmitting the signal of the pressure sensor 5 to the external display device. 7. It is also necessary to provide a power module for supplying power to the pressure sensor and the wireless communication module. Correspondingly, the external display device 7 also needs to be provided with a wireless communication module for receiving the signal of the pressure sensor 5.

Further, the type of the pressure sensor 5 is not specifically limited in the present invention. The pressure sensor 5 may include a semiconductor (such as a silicon wafer) pressure sensor, a piezoelectric pressure sensor, an optical fiber or an optical pressure sensor, and a Perot-type (Fabry-Perot type) pressure sensor, ultrasonic migrator and/or ultrasonic pressure sensor, magnetic pressure sensor, solid state pressure sensor, etc., or any other suitable type of pressure sensor.

Further, it should be avoided as much as possible that the plurality of sensing lines 51 have cross-contact with each other in order to fully ensure the measurement accuracy of the pressure sensor 5. Otherwise, the signals may interfere with each other and affect the accuracy of the measurement results of the pressure sensor 5. Therefore, it is preferable that the portions of the sensing wires 51 located between the proximal-end protective sleeve 3 and the mandrel 4 do not cross each other in the present invention.

More preferably, the portions of the plurality of sensing wires 51 located between the proximal-end protective sleeve 3 and the mandrel 4 may be uniformly distributed along the circumferential direction on the surface of the mandrel 4 as shown in FIG. 2, so that the sensing wires 51 is the farthest distance from each other, and signal interference between each other can be avoided as much as possible. Preferably, as shown in FIG. 3, the sensing wires 51 can also be spirally wound on the surface of the mandrel 4.The flexibility of the guide wire can be better maintained by spirally winding the sensing wires 51 on the mandrel 4, and the sensing wires 51 does not limit the movement of the guide wire when the guidewire is bent.

Further, the pressure sensor 5 can be fixed in the groove 21 of the intermediate ring 2 by any known means in the prior art, for example, it can be adhered by adhesive, or fixed by a physical structure in the groove 21 of the intermediate ring 2, which may be means of engagement or partial wrapping commonly used in the prior art as long as it does not affect the pressure sensor's monitoring of the pressure.

Further, the proximal-end protective sleeve 3 is a heat shrink tube. The plurality of sensing wires 51 are fixed between the proximal-end protective sleeve 3 and the mandrel 4 while the proximal-end protective sleeve 3 shrinks thermally on the surface of the mandrel 4. There is no adhesive between the proximal-end protective sleeve 3 and the mandrel 4, which will not affect the flexibility of the pressure guide wire and the surface of the guide wire will not crack due to bending during use. Thereby, the service life of the guide wire becomes long, the processing of the guide wire is simple, and the surface of the guide wire is smooth, which has no risk of scratching the inner wall of the human body's cavity.

More preferably, the heat shrink tube may be made of a material selected from fluoropolymer, polyurethane, and nylon elastomer. All of the above materials have good bioaffinity. In the present invention, preferably, the heat shrink tube is made of a fluorinated ethylene propylene copolymer (FEP). FEP has low heat shrinking temperature, which can avoid damage to the pressure sensor due to excessive heat shrinking temperature during the production process. Moreover, FEP has a smaller surface friction than other fluoropolymers, and the prepared guide wire has better insertability.

Further, the distal-end protective sleeve 1 may be a spiral metal spring coil or a polymer sleeve, which may be made of one or more materials selected from fluoropolymer, polyurethane or nylon elastomer.

Preferably, the outer diameter of the distal end of the intermediate ring 2 and the proximal end of the distal-end protective sleeve 1 are substantially the same, and the outer diameter of the proximal end of the intermediate ring 2 and the distal end of the proximal-end protective sleeve 3 are substantially the same, that is, the transition among the distal-end protective sleeve 1, the intermediate ring 2 and the proximal-end protective sleeve 3 is smooth, thereby ensuring the flatness and smoothness of the entire surface of the guide wire, and further reducing the possibility of scratching the inner wall of the human body's cavity.

Further, the intermediate ring 2 with the pressure sensor 5 is as shown in FIG. 4. The ratio of length and diameter of the intermediate ring is not particularly limited, which can be adjusted according to the size of the guide wire and the pressure sensor 5. The intermediate ring 2 can be made of a rigid material with a certain hardness, so that the pressure sensor 5 can maintain the original shape when the guide wire is bent, and does not deform as the guide wire is bent, thereby ensuring the measurement accuracy of the pressure sensor 5. More preferably, the length of the groove 21 in the axial direction of the guide wire is greater than the length of the pressure sensor 5, the pressure sensor 5 can be fixed proximal-end protective sleevewithout contacting directly with the proximal-end protective sleeve 3 when the pressure sensor 5 is fixed in the groove 21 of the intermediate ring 2, which can avoid the damage on proximal-end protective sleevethe pressure sensor 5 due to excessive temperature when the proximal-end protective sleeve 3 shrinks thermally on the surface of the mandrel 4.

Preferably, the intermediate ring 2 is a rigid material with a certain hardness. Preferably, the intermediate ring 2 is made of a metal material since the intermediate ring 2 is directly in contact with the heat shrink tube. For example, stainless steel, nickel-titanium alloy, etc. can be used, which can withstand a higher heat shrinking temperature without deformation. More preferably, the intermediate ring 2 may be made of a stainless steel material, which has good developability under X-ray, and helps the operator to accurately locate the position of the pressure sensor 5.

Further, the mandrel 4 is a solid structure made of a metal material, which is not particularly limited. The metal materials commonly used in the field can be selected, for example, various metal materials such as stainless steel (for example, SUS304, SUS303, SUS316, SUS316L, SUS316J1, SUS316J1L, SUS405, SUS430, SUS434, SUS444, SUS429, SUS430F, SUS302, etc.), and alloy materials exhibiting pseudo-elasticity (including super-elastic alloy) can be used, in which super-elastic alloy is preferred. Since the super-elastic alloy is relatively soft and resilient, which is difficult to bend. Therefore, the front end portion of the super-elastic alloy has sufficient bending flexibility and elastic recovery capability when it is used as the guide wire of the mandrel 4, which can improve the insertion performance to a complex, curved lumen, and can obtain better operability. The guide wire does not cause permanent bending deformation due to the excellent elastic recovery ability of the guide wire even if the guide wire undergoes repeated bending deformation, so it is possible to prevent the guide wire from being deteriorated in operability due to bending during use.

Further, the super-elastic alloy is preferably a nickel-titanium alloy. The use of the nickel-titanium alloy as the guide wire of the mandrel 4 has excellent insertion performance, torque transmission property, and further has good operability. At the same time, it has good flexibility and resilience at the distal end, furthermore the trackability and safety of the lumen are improved, and the risk of puncturing the inner wall of the lumen is further reduced.

Further, although the guide wire in the drawings of the present invention is a straight guide wire, the pressure guide wire may also be an elbow guide wire.

Further, the distal end of the proximal-end protective sleeve and/or the distal-end protective sleeve is further wrapped with a functional coating layer. The functional coating layer is a hydrophilic coating, a lubricating coating or a bio-coating. The hydrophilic coating attracts water molecules to form a "gel-like" surface on its surface, reducing resistance when the lead wire is inserted, while further enhancing the biocompatibility of the guide wire and the body tissue. There is no specific limitation on the hydrophilic coating material, and the coating may be formed of a material with low friction resistance. For example, it is preferably made of polyvinyl alcohol, polyvinylpyrrolidone, polyethylene glycol, polyacrylamide, polyacrylic acid, sodium polyacrylate, poly (2-hydroxyethyl methacrylate), maleic anhydride copolymer, ethylene-vinyl alcohol copolymer, 2-methacryloyloxyethyl phosphate choline or its copolymer, (2-hydroxyethyl methacrylate)-styrene block copolymer, one or more of various synthetic polypeptides, collagens, hyaluronates, and one or more of the cellulose-based copolymers.

The type of the lubricating coating is not specifically limited as long as the friction force of the guide wire surface can be reduced and the insertion performance of the guide wire can be improved,. Specifically, the fluoropolymer coating can be selected.

There is no specific limitation on the type of the bio-coating, and a suitable coating layer can be selected according to the needs of the patient's condition. For example, antithrombotic agents such as heparin-quaternary ammonium complexes, antimicrobial agents such as various silver compounds, quaternary ammonium compounds such as benzalkonium chloride, phenol derivatives such as thymol, and antibiotics such as gentamicin, nourishment Ofloxacin, and rifamycin.

The pressure guide wire of the present invention is preferably applied in non-vascular fields, such as urinary, digestive, respiratory, and reproductive systems. Since the non-vascular system lumen is wider than the blood vessel, the pressure sensor 5 can be fully prevented from directly contacting the inner wall of the cavity in the human body under the presence of an endoscope, and it is not necessary to dispose the pressure sensor 5 inside the mandrel 4. Therefore, the size of the guide wire can be reduced, and the measurement accuracy of the pressure sensor 5 can also be improved.

In summary, the present invention can make the pressure sensor directly contact the liquid in the human body's lumen by fixing the pressure sensor in the groove of the intermediate ring, so that the pressure change in the lumen can be monitored accurately and in real time. The sensing wires is wrapped by the proximal-end protective sleeve ,and there is no adhesive between the proximal-end protective sleeve and the mandrel, so the flexibility of the guide wire is good, the guide wire will not crack when bent, and the durability is good. In addition, the entire proximal-end protective sleeve is used to wrap the sensing wires, so that the processing of the guide wire is simple and the outer wall of the guide wire is smooth, which can avoid scratching human organs and has good safety.

## Claims

1. A pressure guide wire, which is configured for use in a non-vascular system, comprising: an insertion portion and an operation portion, wherein the insertion portion comprises a mandrel (4):
a distal-end protective sleeve (1) wrapping the distal end portion of the mandrel (4),
a proximal-end protective sleeve (3) wrapping the proximal end portion of the mandrel (4),
an intermediate sleeve (2) disposed between the distal-end protective sleeve (1) and the proximal-end protective sleeve (3), which is fixedly sleeved on the mandrel (4), and the outer surface of intermediate sleeve (2) is provided with a groove (21),
and, a pressure sensor (5) disposed in the groove (21) of the outer surface of the intermediate sleeve (2) and connected with a plurality of sensing wires (51) for transmitting the signal of the pressure sensor (5), which extend between the proximal-end protective sleeve (3) and the mandrel, mandrel (4), and the other end of which is connected to the operation portion,
the operation portion is connected to the proximal end of the insertion portion, and the operation portion is provided with a connection port connected to the sensing wires (51) or a wireless communication module connected to the sensing wires (51).

2. The pressure guide wire as claimed in claim 1, wherein the portions of the plurality of sensing wires (51) located between the proximal-end protective sleeve (3) and the mandrel (4) do not cross each other.

3. The pressure guide wire as claimed in claim 2, wherein the portions of the plurality of sensing wires (51) located between the proximal-end protective sleeve (3) and the mandrel (4) is uniformly distributed along the circumferential direction on the surface of the mandrel (4).

4. The pressure guide wire as claimed in claim 2, wherein the portions of the plurality of sensing wires (51) located between the proximal-end protective sleeve (3) and the mandrel (4) is spirally wound on the surface of the mandrel (4).

5. The pressure guide wire as claimed in claim 1, wherein the proximal-end protective sleeve (3) is a heat shrink tube, and the heat shrink tube is made of a material selected from fluoropolymer, polyurethane, and nylon elastomer.

6. The pressure guide wire as claimed in claim 5, wherein the heat shrink tube is made of a fluorinated ethylene propylene copolymer.

7. The pressure guide wire as claimed in claim 1, wherein the outer diameter of the distal end of the intermediate sleeve (2) and the proximal end of the distal-end protective sleeve (1) are substantially the same and the outer diameter of the proximal end of the intermediate sleeve (2) and the distal end of the proximal-end protective sleeve (3) are substantially the same.

8. The pressure guide wire as claimed in claim 1, wherein the intermediate sleeve (2) is made of a metal material.

9. The pressure guide wire as claimed in claim 1, wherein the distal end of the proximal-end protective sleeve (3) and/or the distal-end protective sleeve (1) is further wrapped with a functional coating layer, which is a hydrophilic coating, a lubricating coating or a bio-coating.

## Patentansprüche

1. Druckführungsdraht, der zur Verwendung in einem nicht vaskulären System konfiguriert ist, umfassend:
einen Einführungsteil und einen Bedienteil,
wobei der Einführungsteil eine Spitze (4) und einen Drucksensor (5) umfasst, wobei:
der distale Endabschnitt der Spitze (4) von einer distalen Schutzhülle (1) umhüllt ist,
der proximale Endabschnitt der Spitze (4) von einer proximalen Schutzhülle (3) umhüllt ist,
eine zwischen der distalen Schutzhülle (1) und der proximalen Schutzhülle (3) angeordnete Zwischenhülle (2) fest auf der Spitze (4) aufgesteckt ist,
und die Außenfläche der Zwischenhülle mit einer Nut (21) versehen ist,
und der Drucksensor (5) in der Nut (21) der Außenfläche der Zwischenhülle (2) angeordnet und mit einer Vielzahl von Sensordrähten (51) zum Übertragen des Signals des Drucksensors (5) verbunden ist, die sich zwischen dem proximalen Schutzhülle (2) und der Spitze erstrecken,
wobei die Spitze (4) und deren anderes Ende mit dem Bedienteil verbunden ist, wobei der Bedienteil mit dem proximalen Ende des Einführungsteils verbunden ist,
und der Bedienteil mit einem Verbindungsanschluss versehen ist, der mit den Sensordrähten (51) oder mit einem drahtlosen Kommunikationsmodul verbunden ist, das mit den Sensordrähten (51) verbunden ist.

2. Druckführungsdraht nach Anspruch 1, wobei die Teile der Vielzahl von Sensordrähten (51), die sich zwischen dem proximalen Schutzhülle (3) und der Spitze erstrecken, einander nicht kreuzen.

3. Druckführungsdraht nach Anspruch 2, wobei die Teile der Vielzahl von Sensordrähten (51), die zwischen dem proximalen Schutzhülle (5) und der Spitze (4) angeordnet sind, gleichmäßig umlaufend auf der Oberfläche der Spitze (4) verteilt sind.

4. Druckführungsdraht nach Anspruch 2, wobei die Teile der Vielzahl von Sensordrähten (51), die zwischen dem proximalen Schutzhülle (5) und der Spitze (4) angeordnet sind, spiralförmig auf der Oberfläche der Spitze (4) gewickelt sind.

5. Druckführungsdraht nach Anspruch 1, wobei die proximale Schutzhülle (3) ein Wärmeschrumpfschlauch ist, wobei der Wärmeschrumpfschlauch aus einem Material hergestellt ist, das aus Fluorpolymer, Polyurethan und Nylonelastomer ausgewählt wird.

6. Druckführungsdraht nach Anspruch 5, wobei der Wärmeschrumpfschlauch aus dem fluorierten Ethylen-Propylen-Copolymer hergestellt ist.

7. Druckführungsdraht nach Anspruch 1, wobei der Außendurchmesser des distalen Endes der Zwischenhülle (2) und der Außendurchmesser des proximalen Endes der distalen Schutzhülle (1) im Wesentlichen gleich sind und der Außendurchmesser des proximalen Endes der Zwischenhülle (2) und der Außendurchmesser des distalen Endes der proximalen Schutzhülle im Wesentlichen gleich sind.

8. Druckführungsdraht nach Anspruch 1, wobei die Zwischenhülle aus einem metallischen Werkstoff hergestellt ist.

9. Druckführungsdraht nach Anspruch 1, wobei das distale Ende der proximalen Schutzhülle (3) und/oder die distale Schutzhülle (1) ferner mit einer funktionellen Beschichtung beschichtet ist, die eine hydrophobe Beschichtung, eine Gleitbeschichtung oder eine Biobeschichtung ist.

## Revendications

1. Fil de guidage de pression, configuré pour être utilisé dans un système non vasculaire, comprenant :
une partie d'insertion et une partie opérationnelle,
dans lequel la partie d'insertion comprend un mandrin (4) :
un manchon protecteur d'extrémité distale (1) enveloppant la partie d'extrémité distale du mandrin (4),
un manchon protecteur d'extrémité proximale (3) enveloppant la partie d'extrémité proximale du mandrin (4),
un manchon intermédiaire (2) placé entre le manchon protecteur d'extrémité distale (1) et le manchon protecteur d'extrémité proximale (3), qui est emmanché de manière fixe sur le mandrin (4),
et la surface extérieure du manchon intermédiaire est pourvue d'une rainure (21), et, un capteur de pression (5) placé dans la rainure (21) de la surface externe du manchon intermédiaire (2)
et connecté à une pluralité de fils de détection (51) pour transmettre le signal du capteur de pression (5),
qui s'étend entre le manchon de protection de l'extrémité proximale (2) et le mandrin (4),
et dont l'autre extrémité est reliée à la partie opérationnelle,
la partie opérationnelle est reliée à l'extrémité proximale de la partie d'insertion,
et la partie opérationnelle est pourvue d'un port de connexion connecté aux fils de détection (51) ou d'un module de communication sans fil connecté aux fils de détection (51).

2. Le fil de guidage de pression selon la revendication 1, dans laquelle les parties de la pluralité de fils de détection (51) situées entre le manchon de protection d'extrémité proximale (3) et le mandrin ne se croisent pas.

3. Le fil de guidage de pression selon la revendication 2, dans laquelle les parties de la pluralité de fils de détection (51) situées entre le manchon de protection d'extrémité proximale (5) et le mandrin est uniformément distribué le long de la direction circonférentielle sur la surface du mandrin (4).

4. Le fil de guidage de pression selon la revendication 2, dans laquelle les parties de la pluralité de fils de détection (51) situées entre le manchon de protection d'extrémité proximale (3) et le mandrin est enroulé en spirale sur la surface du mandrin (4).

5. Le fil de guidage de pression selon la revendication 1, dans laquelle le manchon de protection de l'extrémité proximale (3) est un tube thermorétractable, et ce dernier est constitué d'un matériau choisi parmi un fluoropolymère, un polyuréthane et un élastomère de nylon.

6. Le fil de guidage sous pression selon la revendication 5, dans laquelle le tube thermorétractable est constitué d'un copolymère éthylène propylène fluoré.

7. Le fil de guidage de pression selon la revendication 1, dans laquelle le diamètre extérieur de l'extrémité distale du manchon intermédiaire et l'extrémité proximale du manchon de protection d'extrémité distale (1) sont sensiblement identiques et le diamètre extérieur de l'extrémité proximale du le manchon intermédiaire et l'extrémité distale du manchon protecteur d'extrémité proximale sont sensiblement les mêmes.

8. Le fil de guidage de pression selon la revendication 1, dans laquelle le manchon intermédiaire est réalisé en un matériau métallique.

9. Le fil de guidage de pression selon la revendication 1, dans laquelle l'extrémité distale du manchon protecteur d'extrémité proximale (3) et/ou du manchon protecteur d'extrémité distale (1) est en outre enveloppée d'une couche de revêtement fonctionnel, qui est un revêtement hydrophile. , un revêtement lubrifiant ou un bio-revêtement.
